(19) Europäisches Patentamt / European Patent Office / Office européen des brevets

(11) **EP 4 227 265 A1**

(12) **EUROPEAN PATENT APPLICATION**
published in accordance with Art. 153(4) EPC

(43) Date of publication:
**16.08.2023   Bulletin 2023/33**

(21) Application number: **22859910.6**

(22) Date of filing: **29.04.2022**

(51) International Patent Classification (IPC):
**C01B 39/44** $^{(2006.01)}$    **B01J 29/67** $^{(2006.01)}$
**C07C 5/27** $^{(2006.01)}$

(52) Cooperative Patent Classification (CPC):
**B01J 29/65; B01J 29/67; C01B 39/40; C01B 39/44; C07C 5/27; C07C 11/09; C07C 11/10;** Y02P 20/52

(86) International application number:
**PCT/CN2022/090246**

(87) International publication number:
**WO 2023/024560 (02.03.2023 Gazette 2023/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.08.2021   CN 202110981905**

(71) Applicant: **PetroChina Company Limited**
**Dongcheng District**
**Beijing 100007 (CN)**

(72) Inventors:
• **ZHANG, Xuejun**
  **Beijing 100724 (CN)**
• **GAO, Zhuoran**
  **Beijing 100724 (CN)**
• **HU, Yaqiong**
  **Beijing 100724 (CN)**
• **KANG, Hongmin**
  **Beijing 100724 (CN)**
• **MA, Jianbo**
  **Beijing 100724 (CN)**

(74) Representative: **Slingsby Partners LLP**
**1 Kingsway**
**London WC2B 6AN (GB)**

(54) **ZSM-35 MOLECULAR SIEVE AND PREPARATION METHOD THEREFOR, ISOMERIZATION CATALYST AND PREPARATION METHOD THEREFOR, AND ISOMERIZATION METHOD**

(57)    The present disclosure provides a ZSM-35 molecular sieve and a preparation method therefor, an isomerization catalyst and a preparation method therefor, and an isomerization method. The preparation method for a ZSM-35 molecular sieve comprises: mixing a silicon source, an aluminum source, an alkali, a template agent and water, then adding a polyacrylamide thereto, and performing crystallization on same twice to obtain a ZSM-35 molecular sieve. The present disclosure further provides an isomerization catalyst prepared from the ZSM-35 molecular sieve and a preparation method therefor, and an isomerization method.

Fig. 1

EP 4 227 265 A1

**Description**

Technology Field

The present invention relates to the technical field of catalytic chemistry, in particular to a ZSM-35 molecular sieve and its production method, and to an isomerization catalyst suitable for the isomerization of light olefins and its production method, and an isomerization method.

Background Art

ZSM-35 molecular sieve is a molecular sieve with FER topology, which has a two-dimensional pore system of vertically crossed 10-membered ring-8-membered ring, in which the pore size of 10-membered ring is $0.42 \times 0.54$ nm; and the pore size of 8-membered ring is $0.35 \times 0.48$ nm. ZSM-35 molecular sieve is widely used in catalytic reactions for hydrocarbon conversion, such as isomerization, aromatization, polymerization and cracking of straight-chain olefins, due to its unique vertically crossed two-dimensional pore structure.

The existing skeletal isomerization catalysts for olefins have high reaction temperatures, with starting temperatures usually above 350°C. Moreover, the selectivity of the catalysts at the early stage of the reaction is relatively poor, with more cracking products.

Summary of the invention

In view of above problems, the present invention is to provide a ZSM-35 molecular sieve and its production method, and an isomerization catalyst and its production method and an isomerization method. The isomerization catalyst has high isomerization activity and selectivity for light olefins and can convert straight-chain olefins to branched olefins at a lower temperature for an isomerization reaction.

To achieve the above purpose, the present invention provides a method for producing a ZSM-35 molecular sieve, comprising: mixing a silicon source, an aluminum source, an alkali, and a template agent with water to form a mixed solution, adding polyacrylamide to the mixed solution to form a feedstock solution, and crystallizing the feedstock solution at 140-160°C for 12-24 h and then at 120-140°C for 48-72 h to obtain the ZSM-35 molecular sieve, wherein the template agent comprises pyrrolidine.

In the production method described above, a ZSM-35 molecular sieve with high crystallinity and small grains can be obtained by selecting pyrrolidine as the template agent, adding polyacrylamide and using two-stage controlling for crystallization temperature.

In the production method described above, the temperatures of the first stage crystallization (crystallization at 140-160°C for 12-24 h) and the second stage crystallization (crystallization at 120-140°C for 48-72 h) are different. Generally, the temperature of the first stage crystallization is higher than that of the second stage crystallization. For example, when the temperature of the first stage crystallization is 140°C, the temperature of the second stage crystallization is not 140°C, but may be 120°C, 130°C, etc.

In the production method described above, adding polyacrylamide facilitates the homogeneous dispersion of the components in the feedstock solution and facilitates to obtain a ZSM-35 molecular sieve having a small size. In some specific embodiments, the amount by weight of the polyacrylamide is generally controlled to be 0.1-1 wt% of the mixed solution. The polyacrylamide may have a weight-average molecular weight of 3-10 million.

According to a specific embodiment of the present invention, in the production method described above, the silicon source, the aluminum source, the alkali, pyrrolidine (Py) and water may be mixed in molar ratios of $SiO_2/Al_2O_3$=20-110, $H_2O/SiO_2$=10-80, $NaOH/SiO_2$=0.1-1.0, and $Py/SiO_2$=0.1-1.0. Preferably, the silicon source, the aluminum source, the alkali, pyrrolidine and water are mixed in molar ratios of $SiO_2/Al_2O_3$=30-90, $H_2O/SiO_2$=20-50, $NaOH/SiO_2$=0.2-0.5, and $Py/SiO_2$=0.2-0.5.

In the production method described above, the silicon source is generally a commonly used silicon source, which may comprise, for example, silica sol, solid silica gel, white carbon black, etc.

In the production method described above, the aluminum source is generally a commonly used silicon source, which may comprise, for example, aluminum sulfate, aluminum oxide, sodium meta-aluminate, etc.

In the production method described above, the alkali is generally a commonly used alkali, which may comprise, for example, sodium hydroxide, etc.

The present invention further provides a ZSM-35 molecular sieve obtained by the production method described above. In a specific embodiment, the ZSM-35 molecular sieve obtained by the above method is generally a lamellar crystal with small grains and high crystallinity, having a grain thickness of less than or equal to 50 nm, and a grain length and/or a grain width of less than or equal to 500 nm, and a crystallinity of greater than equal to 120%. For example, the above ZSM-35 molecular sieve with small grains and high crystallinity can achieve a grain thickness of less than 50 nm, a grain length and/or width of less than or equal to 200 nm, and a crystallinity of greater than or equal to 140%.

The present invention further provides a method for producing an isomerization catalyst, comprising: subjecting a template agent-containing ZSM-35 molecular sieve to ammonium salt exchange, then mixing it with water, a binder, an acid and an extrusion aid to form a feedstock, and then extruding, drying, and baking the mixture to obtain the isomerization catalyst, wherein the template agent-containing ZSM-35 molecular sieve is not subjected to a process of removing the template agent prior to the extruding, and is not subjected to a baking process after the ammonium salt exchange (i.e., the feedstock containing the ZSM-35 molecular sieve is not baked after the ammonium salt exchange).

The inventors have found that the activity and selectivity of isomerization catalysts can be effectively enhanced by reducing the grain size and increasing the crystallinity of the molecular sieve for isomerization catalysts. The conventional production process of a molecular sieve catalyst usually includes processes involving baking, such as the removal of template agent from the raw molecular sieve powder by baking, ammonium decomposition by baking after the exchange of molecular sieve, and activation by baking after the formation of molecular sieve catalyst. It is generally believed that the removal of template agent by baking can form a regular cavity skeleton structure, which in turn becomes an endocrystalline space for adsorption and catalysis, and is conducive to the performance of the molecular sieve catalyst. The inventors have found that both the removal of template agent by heat treatment such as baking and the baking after ammonium salt exchange would significantly reduce the crystallinity of the molecular sieve, and that the baking process to remove the molecular sieve and the baking process after ammonium salt exchange also degrade the performance of the catalyst. Therefore, by synthesizing a ZSM-35 molecular sieve with high crystallinity and small particle size and reducing the numbers of baking process in the catalyst production, the present invention can maintain the high crystallinity of the ZSM-35 molecular sieve during in the production of an isomerization catalyst, and thus improve the activity and selectivity of the isomerization catalyst.

In a specific embodiment of the present invention, the acid may comprise an organic acid and/or an inorganic acid. The organic acid may comprise acetic acid and/or citric acid. The inorganic acid may comprise nitric acid.

In a specific embodiment of the present invention, the binder may be a commonly used binder, which may comprise, for example, one or more of alumina sol, alumina, and pseudo-boehmite. Preferably, the binder comprises pseudo-boehmite.

In a specific embodiment of the present invention, the extrusion aid may be a commonly used aid, which may comprise, for example, Sesbania powder and/or methyl cellulose.

In a specific embodiment of the present invention, the ammonium salt may be ammonium nitrate. The ammonium salt is generally in the form of a solution, and the mass concentration of the ammonium salt solution is generally 1-10%. For example, the ZSM-35 molecular sieve can be exchanged with an ammonium nitrate solution having a mass concentration of 1-10%, where the number of exchanges is not limited. No baking treatment is performed after the exchange and before the formation, and the ammonium-based molecular sieve is not converted to a hydrogen-based molecular sieve.

In a specific embodiment of the present invention, the template agent-containing ZSM-35 molecular sieve is generally a ZSM-35 molecular sieve made by adding and retaining the template agent, such as a ZSM-35 molecular sieve obtained by using the method for producing a ZSM-35 molecular sieve described above.

In a specific embodiment of the present invention, if the temperature of the baking performed after extrusion molding is too low, the decomposition of organic or inorganic substances will be incomplete; if the temperature is too high, it may cause excessive loss of crystallinity of the molecular sieve. As a result, the baking temperature is generally controlled at 530-550°C. The baking time can be adjusted as a function of the baking temperature, for example, 4-8 h.

In a specific embodiment of the present invention, the production method described above can comprise a process of exchanging the ZSM-35 molecular sieve with ammonium salt, prior to washing with water and drying at a temperature of generally controlled at 120-140°C.

In a specific embodiment of the present invention, the production method described above can comprise a process of drying the extruded product preferably at a temperature of 120-140°C.

The present invention further provides an isomerization catalyst obtained by the method for producing an isomerization catalyst described above.

In the isomerization catalyst described above, the mass of the template agent-containing molecular sieve generally accounts for 80% or more (preferably, 90% or more) of the total mass of the isomerization catalyst, to ensure maintaining the conversion by the isomerization catalyst at a high level and stable over a long period of time. In some specific embodiments, the mass of the binder generally accounts for 5-20% of the total mass of the isomerization catalyst.

The present invention further provides an isomerization method, comprising: catalyzing an isomerization reaction of light straight-chain olefins of C6 or less using the isomerization catalyst described above to convert them into isomeric light branched olefins of C6 or less, under a non-hydrogen exposure condition (i.e., a gaseous environment without hydrogen).

In a specific embodiment of the present invention, the isomerization reaction of the olefins is carried out at a pressure of 0.05-0.5 MPa, an LHSV of 2.0-6.0 h$^{-1}$ and a temperature of 240-420°C; preferably, the isomerization reaction is carried out at a pressure of 0.1-0.3 MPa, an LHSV of 3.0-5.0 h$^{-1}$ and a temperature of 260-385°C.

In a specific embodiment of the present invention, the light straight-chain olefins may be n-butene, or n-pentene, etc., or a mixture rich in (≮15% by volume) n-butene and/or n-pentene, etherified C4 components, and etherified C5 components, etc., for example, etherified C4 components, etherified C5 components, etc., from refineries and ethylene plants.

In a specific embodiment of the present invention, the isomerization reaction can be carried out in a fixed bed reactor, or in a moving bed, fluidized bed, etc., and can be carried out at an atmospheric pressure or higher pressure.

The beneficial effect of the present invention lies in that the isomerization catalyst provided by the present invention can be applied to catalyze the isomerization of light straight-chain olefins, and can catalyze the isomerization reaction at a lower temperature, while exhibiting high catalytic activity and selectivity.

Brief Description of the Drawings

Fig. 1 shows the results for characterizing the morphological structure of the ZSM-35 molecular sieve produced in Comparative Example 1.

Fig. 2 shows the results for characterizing the morphological structure of the ZSM-35 molecular sieve produced in Example 1.

Fig. 3 shows the process flow diagram of the skeletal isomerization of light olefins in Examples 10 and 11.

Detailed Description of Preferred Embodiments

In order to have a clearer understanding of the technical features, objectives and beneficial effects of the present invention, the technical solutions of the present invention are now described in detail in the following, which, however, should not be understood as limiting the implementable scope of the present invention.

The weight-average molecular weight of polyacrylamide used in the following Examples and Comparative Examples is 3 million.

The crystallinity of the molecular sieve was measured using a smartlab type X-ray diffractometer of Rigaku Co. Ltd., with CuKα line as the radiation source, a tube voltage of 40 KV, a tube current of 50 mA, a scan rate of 5°/min, and a scan range of 2θ=5-85°. The crystallinity was obtained using the measurements of intensities of characteristic peaks (9.4°, 22.4°, 22.7°, 23.3°, 23.7°, 24.5°, 25.3°) by a common calculation method.

The SEM characterization was carried out using a 200F type field emission scanning electron microscope from Quanta chrome Co. Ltd., with a test voltage of 200 KV. The grain size of the molecular sieve was measured by the 200F type field emission scanning electron microscope, and the results were averaged for the different grains of molecular sieve measured.

There are two important indicators for evaluating the performance of the skeletal isomerization catalyst of olefins: (1) the conversion of n-pentene (or n-butene), and the selectivity for isopentene (or isobutene), which are defined as follows:

$$conversion(\text{n - }pentene)\frac{\text{n - pentene content in feedstock}-\text{n - pentene content in product}}{\text{n - pentene content in feedstock}}\times100\%$$

$$\text{Selectivity (iso}pentene)\frac{\text{isopentene content in product}-\text{isopentene content in feedstock}}{\text{n - pentene content in feedstock}-\text{n - pentene content in product}}\times100\%$$

$$conversion(\text{n - bu}tene)\frac{\text{n - butene content in feedstock}-\text{n - butene content in product}}{\text{n - butene content in feedstock}}\times100\%$$

$$\text{Selectivity (isobu}tene)\frac{\text{isobutene content in product}-\text{isobutene content in feedstock}}{\text{n - butene content in feedstock}-\text{n - butene content in product}}\times100\%$$

Comparative Example 1

This Comparative Example provides a method for producing a ZSM-35 molecular sieve, which comprises the following processes.

Silica sol (25.0 wt% of $SiO_2$), aluminum sulfate (99.0%), sodium hydroxide and pyrrolidine (99.0%) were added to deionized water in the molar ratio of $SiO_2/Al_2O_3$=30, $H_2O/SiO_2$=50, $NaOH/SiO_2$=0.5, $Py/SiO_2$=0.5, stirred well, and crystallized at 150°C for 60 h. After crystallization, it was filtered and washed with deionized water, and then dried at 120°C for 4 h to obtain the ZSM-35 molecular sieve.

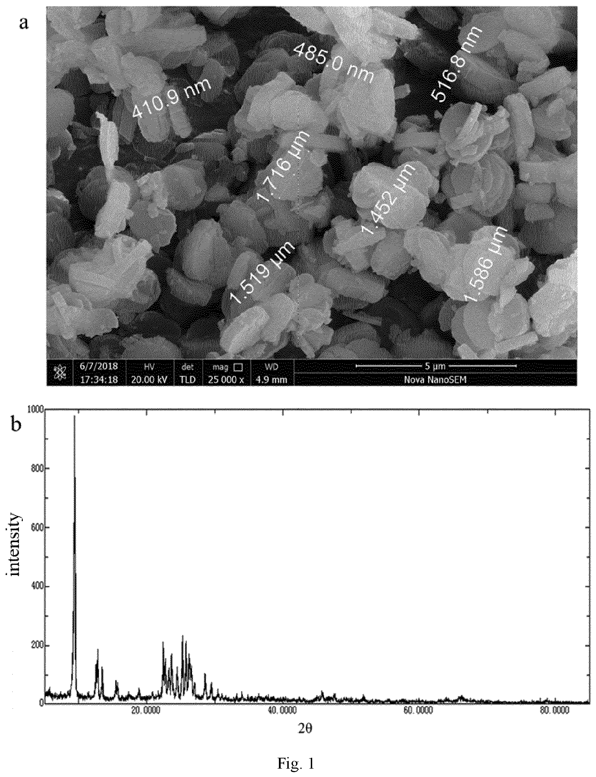

The ZSM-35 molecular sieve was analyzed by scanning electron microscopy (SEM) and X-ray diffraction (XRD), and the SEM and XRD results are shown in Fig. 1 (a) and (b). The ZSM-35 molecular sieve was measured and calculated to have a grain thickness of 471 nm, a grain length or grain width of 1568 nm, and a crystallinity of 105%.

Comparative Example 2

This Comparative Example provides a method for producing a ZSM-35 molecular sieve, which comprises the following processes.

Silica sol (25.0 wt% of $SiO_2$), aluminum sulfate (99.0%), sodium hydroxide and pyrrolidine (99.0%) were added to deionized water in the molar ratio of $SiO_2/Al_2O_3$=50, $H_2O/SiO_2$=40, $NaOH/SiO_2$=0.3, $Py/SiO_2$=0.3, stirred well, and crystallized at 150°C for 72 h. After crystallization, it was filtered and washed with deionized water, and then dried at 120°C for 4 h to obtain raw powders of the ZSM-35 molecular sieve.

The ZSM-35 molecular sieve was analyzed by scanning electron microscopy (SEM) and X-ray diffraction (XRD). The ZSM-35 molecular sieve was measured and calculated to have a grain thickness of 421 nm, a grain length or grain width of 1652 nm, and a crystallinity of 100%.

Comparative Example 3

This Comparative Example provides a method for producing a ZSM-35 molecular sieve, which comprises the following processes.

Silica sol (25.0 wt% of $SiO_2$), aluminum sulfate (99.0%), sodium hydroxide and pyrrolidine (99.0%) were added to deionized water in the molar ratio of $SiO_2/Al_2O_3$=70, $H_2O/SiO_2$=30, $NaOH/SiO_2$=0.2, $Py/SiO_2$=0.2, stirred well, and crystallized at 160°C for 72 h. After crystallization, it was filtered and washed with deionized water, and then dried at 120°C for 4 h to obtain the ZSM-35 molecular sieve.

The ZSM-35 molecular sieve was analyzed by scanning electron microscopy (SEM) and X-ray diffraction (XRD). The ZSM-35 molecular sieve was measured and calculated to have a grain thickness of 485 nm, a grain length or grain width of 1698 nm, and a crystallinity of 95%.

Comparative Example 4

This Comparative Example provides a method for producing an isomerization catalyst, which comprises the following processes.

1000 g of the ZSM-35 molecular sieve (which retains the template agent) produced in the Comparative Example 1 was exchanged 3 times with 5% ammonium nitrate solution at a liquid-solid ratio of 5:1, and then the exchanged ZSM-35 molecular sieve was washed 3 times with deionized water at a liquid-solid ratio of 5:1. After filtration, it was dried at 120°C for 4 h. All the dried ZSM-35 molecular sieve was mixed uniformly with 100 g of pseudo-boehmite (specific surface area: 288 $m^2$/g, dry basis: 68%) and 30 g of Sesbania powder. 150 g of citric acid was added to 850 g of deionized water and stirred well. After that, the formed citric acid solution was added to the mixture, kneaded with a kneading machine, and then extruded with an extruder. After drying in an oven at 120°C for 4 h, it was transferred to a muffle furnace where it was heated up to 550°C for 4 h and kept at the constant temperature for 4 h. At the end of baking, a skeletal isomerization catalyst (A) for light olefins was produced.

Comparative Example 5

This Comparative Example provides a method for producing an isomerization catalyst, which comprises the following processes.

1000 g of the ZSM-35 molecular sieve produced in the Comparative Example 2 was exchanged 3 times with 5% ammonium nitrate solution according to a liquid-solid ratio of 5:1, and then the exchanged ZSM-35 molecular sieve was washed 3 times with deionized water at a liquid-solid ratio of 2:1. After filtration, it was dried at 120-140°C for 4 h. All the dried ZSM-35 molecular sieve was mixed uniformly with 100 g of pseudo-boehmite (specific surface area: 288

$m^2/g$, dry basis: 68%) and 50 g of methyl cellulose to obtain a mixture. 50 g of nitric acid was added to 900 g of deionized water and stirred well. After that, the formed nitric acid solution was added to the mixture, kneaded with a kneading machine, and then extruded with an extruder. After drying in an oven at 140°C for 4 h, it was transferred to a muffle furnace where it was heated up to 550°C for 4 h and kept at the constant temperature for 4 h. At the end of baking, a skeletal isomerization catalyst (B) for light olefins was produced.

Comparative Example 6

This Comparative Example provides a method for producing an isomerization catalyst, which comprises the following processes.

1000 g of the ZSM-35 molecular sieve produced in the Comparative Example 3 was exchanged 3 times with 5% ammonium nitrate solution according to a liquid-solid ratio of 5:1, and then the exchanged ZSM-35 molecular sieve was washed 3 times with deionized water at a liquid-solid ratio of 5:1. After filtration, it was dried at 120°C for 4 h. All the dried ZSM-35 molecular sieve was mixed uniformly with 90 g of alumina (specific surface area: 212 $m^2/g$, dry basis: 75%) and 30 g of Sesbania powder. 100 g of acetic acid was added to 850 g of deionized water and stirred well. After that, the formed acetic acid solution was added to the mixture, kneaded with a kneading machine, and then extruded with an extruder. After drying in an oven at 120°C for 4 h, it was transferred to a muffle furnace where it was heated up to 550°C for 4 h and kept at the constant temperature for 4 h. At the end of baking, a skeletal isomerization catalyst (C) for light olefins was produced.

Example 1

This Example provides a method for producing a ZSM-35 molecular sieve, which comprises the following processes.

Silica sol (25.0 wt% of $SiO_2$), aluminum sulfate (99.0%), sodium hydroxide and pyrrolidine (99.0%) were added to deionized water in the molar ratio of $SiO_2/Al_2O_3$=30, $H_2O/SiO_2$=50, $NaOH/SiO_2$=0.5, $Py/SiO_2$=0.5, and stirred well to form a mixed solution. To the mixed solution, 0.20% by weight of polyacrylamide was added, crystallized at 150°C for 12 h, and then crystallized at 150°C for 60 h. After crystallization, it was filtered and washed with deionized water, and then dried at 120°C for 4 h to obtain the ZSM-35 molecular sieve.

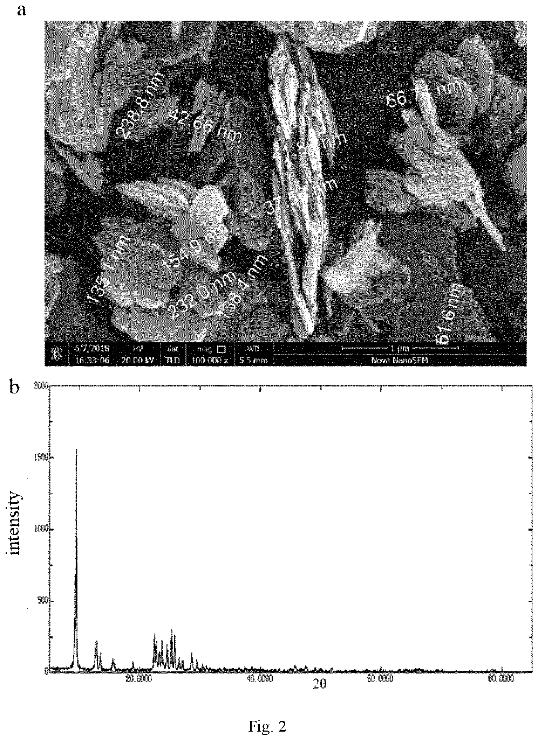

This ZSM-35 molecular sieve was analyzed by scanning electron microscopy (SEM) and X-ray diffraction (XRD), and the SEM and XRD results are shown in Fig. 2 (a) and (b). The ZSM-35 molecular sieve was measured and calculated to have a grain thickness of 47 nm, a grain length or grain width of 177 nm, and a crystallinity of 142%.

Example 2

This Example provides a method for producing a ZSM-35 molecular sieve, which comprises the following processes.

Silica sol (25.0 wt% of $SiO_2$), aluminum sulfate (99.0%), sodium hydroxide and pyrrolidine (99.0%) were added to deionized water in the molar ratio of $SiO_2/Al_2O_3$=50, $H_2O/SiO_2$=40, $NaOH/SiO_2$=0.3, $Py/SiO_2$=0.3, and stirred well to form a mixed solution. To the mixed solution, 0.5% by weight of polyacrylamide was added, crystallized at 150°C for 18 h, and then crystallized at 130°C for 72 h. After crystallization, it was filtered and washed with deionized water, and then dried at 120°C for 4 h to obtain the ZSM-35 molecular sieve.

This ZSM-35 molecular sieve was analyzed by scanning electron microscopy (SEM) and X-ray diffraction (XRD). The ZSM-35 molecular sieve was measured and calculated to have a grain thickness of 42 nm, a grain length or grain width of 186 nm, and a crystallinity of 145%.

Example 3

This Example provides a method for producing a ZSM-35 molecular sieve, which comprises the following processes.

Silica sol (25.0 wt% of $SiO_2$), aluminum sulfate (99.0%), sodium hydroxide and pyrrolidine (99.0%) were added to deionized water in the molar ratio of $SiO_2/Al_2O_3$=70, $H_2O/SiO_2$=30, $NaOH/SiO_2$=0.2, $Py/SiO_2$=0.2, and stirred well to form a mixed solution. To the mixed solution, 0.20% by weight of polyacrylamide was added, crystallized at 160°C for 24 h, and then crystallized at 140°C for 60 h. After crystallization, it was filtered and washed with deionized water, and then dried at 120°C for 4 h to obtain the ZSM-35 molecular sieve.

This ZSM-35 molecular sieve was analyzed by scanning electron microscopy (SEM) and X-ray diffraction (XRD). The ZSM-35 molecular sieve was measured and calculated to have a grain thickness of 47 nm, a grain length or grain width of 312 nm, and a crystallinity of 124%.

Example 4

This Example provides a method for producing an isomerization catalyst, which comprises the following processes.

1000 g of the template agent-containing ZSM-35 molecular sieve with small grains and high crystallinity produced in the Example 1 was baked at 530°C for 8 h to remove the template agent, and then exchanged 3 times with 5% ammonium nitrate solution at a liquid-solid ratio of 5:1. The exchanged ZSM-35 molecular sieve was washed 3 times with deionized water at a liquid-solid ratio of 5:1. After filtration, it was dried at 120°C for 4 h and baked at 500°C for 4 h. All the dried ZSM-35 molecular sieve was mixed uniformly with 100 g of pseudo-boehmite (specific surface area: 288 $m^2/g$, dry basis: 68%) and 30 g of Sesbania powder. 150 g of citric acid was added to 850 g of deionized water and stirred well. After that, it was added to the mixture, kneaded with a kneading machine, and then extruded with an extruder. After drying in an oven at 120°C for 4 h, it was transferred to a muffle furnace where it was heated up to 500°C for 4 h and kept at the constant temperature for 4 h. At the end of baking, a skeletal isomerization catalyst (D) for light olefins was produced.

Example 5

This Example provides a method for producing an isomerization catalyst, which comprises the following processes.

1000 g of the template agent-containing ZSM-35 molecular sieve with small grains and high crystallinity in the Example 2 was baked at 530°C for 8 h to remove the template agent, and then exchanged 3 times with 5% ammonium nitrate solution at a liquid-solid ratio of 5:1. The exchanged ZSM-35 molecular sieve was washed 3 times with deionized water at a liquid-solid ratio of 2:1. After filtration, it was dried at 120-140°C for 4 h and baked at 500°C for 4 h. All the dried ZSM-35 molecular sieve was mixed uniformly with 100 g of pseudo-boehmite (specific surface area: 288 $m^2/g$, dry basis: 68%) and 50 g of methyl cellulose to obtain a mixture. 50 g of nitric acid was added to 900 g of deionized water and stirred well. After that, it was added to the mixture, kneaded with a kneading machine, and then extruded with an extruder. After drying in an oven at 140°C for 4 h, it was transferred to a muffle furnace where it was heated up to 500°C for 4 h and kept at the constant temperature for 4 h. At the end of baking, a skeletal isomerization catalyst (E) for light olefins was produced.

Example 6

This Example provides a method for producing an isomerization catalyst, which comprises the following processes.

1000 g of the template agent-containing ZSM-35 molecular sieve with small grains and high crystallinity in the Example 3 was baked at 530°C for 8 h to remove the template agent, and then exchanged 3 times with 5% ammonium nitrate solution at a liquid-solid ratio of 5:1. The exchanged ZSM-35 molecular sieve was washed 3 times with deionized water at a liquid-solid ratio of 5:1. After filtration, it was dried at 120°C for 4 h and baked at 500°C for 4 h. All the dried ZSM-35 molecular sieve was mixed uniformly with 90 g of alumina (specific surface area: 212 $m^2/g$, dry basis: 75%) and 30 g of Sesbania powder. 100 g of acetic acid was added to 850 g of deionized water and stirred well. After that, it was added to the mixture, kneaded with a kneading machine, and then extruded with an extruder. After drying in an oven at 120°C for 4 h, it was transferred to a muffle furnace where it was heated up to 500°C for 4 h and kept at the constant temperature for 4 h. At the end of baking, a skeletal isomerization catalyst (F) for light olefins was produced.

Example 7

This Example provides a method for producing an isomerization catalyst, which comprises the following processes.

1000 g of the template agent-containing ZSM-35 molecular sieve with small grains and high crystallinity in the Example 1 was exchanged 3 times with 5% ammonium nitrate solution at a liquid-solid ratio of 5:1, and the exchanged ZSM-35 molecular sieve was then washed 3 times with deionized water at a liquid-solid ratio of 5:1. After filtration, it was dried at 120°C for 4 h. All the dried ZSM-35 molecular sieve was mixed uniformly with 100 g of pseudo-boehmite (specific surface area: 288 $m^2/g$, dry basis: 68%) and 30 g of Sesbania powder. 150 g of citric acid was added to 850 g of deionized water and stirred well. After that, it was added to the mixture, kneaded with a kneading machine, and then extruded with an extruder. After drying in an oven at 120°C for 4 h, it was transferred to a muffle furnace where it was heated up to 550°C for 4 h and kept at the constant temperature for 4 h. At the end of baking, a skeletal isomerization catalyst (G) for light olefins was produced.

Example 8

This Example provides a method for producing an isomerization catalyst, which comprises the following processes.

1000 g of the template agent-containing ZSM-35 molecular sieve with small grains and high crystallinity in the Example 2 was exchanged 3 times with 10% ammonium nitrate solution at a liquid-solid ratio of 5:1, and the exchanged ZSM-35 molecular sieve was then washed 3 times with deionized water at a liquid-solid ratio of 2:1. After filtration, it was dried at 120-140°C for 4 h. All the dried ZSM-35 molecular sieve was mixed uniformly with 100 g of pseudo-boehmite (specific surface area: 288 m$^2$/g, dry basis: 68%) and 50 g of methyl cellulose to obtain a mixture. 50 g of nitric acid was added to 900 g of deionized water and stirred well. After that, it was added to the mixture, kneaded with a kneading machine, and then extruded with an extruder. After drying in an oven at 140°C for 4 h, it was transferred to a muffle furnace where it was heated up to 550°C for 4 h and kept at the constant temperature for 4 h. At the end of baking, a skeletal isomerization catalyst (H) for light olefins was produced.

Example 9

This Example provides a method for producing an isomerization catalyst, which comprises the following processes.

1000 g of the template agent-containing ZSM-35 molecular sieve with small grains and high crystallinity in the Example 3 was exchanged 3 times with 5% ammonium nitrate solution at a liquid-solid ratio of 5:1, and the exchanged ZSM-35 molecular sieve was then washed 3 times with deionized water at a liquid-solid ratio of 5:1. After filtration, it was dried at 120°C for 4 h. All the dried ZSM-35 molecular sieve was mixed uniformly with 90 g of alumina (specific surface area: 212 m$^2$/g, dry basis: 75%) and 30 g of Sesbania powder. 100 g of acetic acid was added to 850 g of deionized water and stirred well. After that, it was added to the mixture, kneaded with a kneading machine, and then extruded with an extruder. After drying in an oven at 120°C for 4 h, it was transferred to a muffle furnace where it was heated up to 550°C for 4 h and kept at the constant temperature for 4 h. At the end of baking, a skeletal isomerization catalyst (I) for light olefins was produced.

Example 10

In this example, isomerization experiments were carried out using the isomerization catalysts produced in Comparative Examples 4-6 and Examples 4-9, respectively, with refinery etherified C5 light hydrocarbons as the experimental feedstock. The specific composition is shown in Table 1.

Table 1

| components | content, m% |
| --- | --- |
| 1-pentene | 4.27 |
| 2-methyl-1-butene | 0.82 |
| trans-2-pentene+cis-2-pentene | 18.72 |
| 2-methyl-2-butene | 8.14 |
| isopentane | 42.19 |
| n-pentane | 6.44 |
| others | 5.12 |

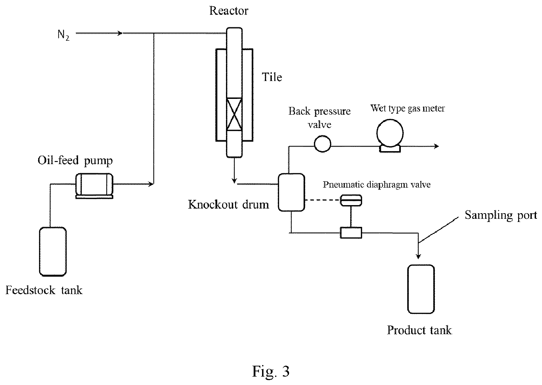

The experiment was carried out in a 20 ml atmospheric pressure reactor with an isothermal fixed bed reactor to pass the product through the process once. The experimental process flow is shown in Fig. 3.
The experimental process was as follows:

1. The isomerization catalyst was crushed into 20-30 mesh particles with a loading volume of 20 ml and filled in the constant temperature section of the reactor. The upper and lower part of the catalyst was loaded with 20-30 mesh quartz sand. After the completion of filling, the reactor was connected to the system. Nitrogen gas was introduced for gas tightness test, and the gas-tight pressure was gradually increased to 1.0 MPa. Once the pressure drop was not more than 0.1 MPa after standing for 2 hours, the device was determined to be gas-tight and qualified.

2. Etherified C5 light hydrocarbons were subjected to a skeletal isomerization experiment for light olefins under the conditions of a pressure of 0.1MPa, an LHSV of 3.0 h$^{-1}$ and a temperature of 260°C, and then the hydrocarbon composition of the product was analyzed.

The above experiments were carried out with isomerization catalysts from Comparative Examples 4-6 and Examples 4-9, respectively, and the obtained results are summarized in Table 2.

Table 2

| Items | | Comp. Ex. 4 | Comp. Ex. 5 | Comp. Ex. 6 | Example 4 | Example 5 | Example 6 | Example 7 | Example 8 | Example 9 |
|---|---|---|---|---|---|---|---|---|---|---|
| Catalyst No. | | A | B | C | D | E | F | G | H | I |
| Convention of n-pentene, % | | 45.1 | 43.2 | 42.5 | 63.5 | 61.2 | 59.6 | 66.2 | 65.8 | 65.2 |
| Selectivity for isopentene, % | | 95.2 | 94.5 | 95.6 | 96.1 | 96.3 | 96.5 | 97.1 | 96.8 | 97.3 |

Example 11

In this example, isomerization experiments were carried out using the isomerization catalysts produced in Comparative Examples 4-6 and Examples 4-9, respectively, with refinery etherified C4/C5 light hydrocarbons as the experimental feedstock. The specific composition is shown in Table 3.

Table 3

| components | content, m% |
|---|---|
| 1-butene | 21.75 |
| trans-2-butene | 22.33 |
| cis-2-butene | 18.06 |
| isobutene | 1.16 |
| isopentane | 28.15 |
| n-pentane | 6.48 |
| others | 2.12 |

The experimental process flow is shown in Fig. 3.
The experimental process was as follows:

1. The isomerization catalyst was crushed into 20-30 mesh particles with a loading volume of 20 ml and filled in the constant temperature section of the reactor. The upper and lower part of the catalyst was loaded with 20-30 mesh quartz sand. After the completion of filling, the reactor was connected to the system. Nitrogen gas was introduced for gas tightness test, and the gas-tight pressure was gradually increased to 1.0 MPa. Once the pressure drop was not more than 0.1 MPa after standing for 2 hours, the device was determined to be gas-tight and qualified.

2. Etherified C4 light hydrocarbons were subjected to a skeletal isomerization experiment for light olefins under the conditions of a pressure of 0.3 MPa, an LHSV of 5.0 $h^{-1}$ and a temperature of 320°C, and then the hydrocarbon composition of the product was analyzed.

The above experiments were carried out with isomerization catalysts from Comparative Examples 4-6 and Examples 4-9, respectively, and the obtained results are summarized in Table 4.

Table 4

| Items | Comp. Ex. 4 | Comp. Ex. 5 | Comp. Ex. 6 | Example 4 | Example 5 | Example 6 | Example 7 | Example 8 | Example 9 |
|---|---|---|---|---|---|---|---|---|---|
| Catalyst No. | A | B | C | D | E | F | G | H | I |
| Convention of n-butene, % | 33.8 | 32.6 | 30.8 | 38.6 | 37.2 | 36.3 | 42.5 | 41.2 | 39.1 |
| Selectivity for isobutene, % | 86.1 | 87.2 | 86.5 | 86.9 | 87.5 | 87.2 | 88.3 | 87.9 | 88.5 |

The differences between the molecular sieve production process of Comparative Examples 1-3 and that of Examples 1-3 are as follows: a traditional molecular sieve synthesis method was used in Comparative Examples 1-3, in which no polyacrylamide was added and the crystallization was carried out only once, and the synthesized molecular sieves had larger grain sizes, grain thicknesses greater than 400 nm, grain lengths or widths greater than 1500 nm, and lower crystallinities, while the molecular sieve production method of the present invention was used in Examples 1-3, in which polyacrylamide was added and crystallization was carried out twice, and the synthesized molecular sieves had smaller grain sizes and higher crystallinities. Specifically, the molecular sieves synthesized in Examples 1-3 had grain thicknesses less than 50 nm, grain lengths or widths less than 500 nm, and crystallinities larger than 120%; further, the molecular sieves synthesized in Examples 1-2 had grain thicknesses less than 50 nm, grain lengths or widths less than 200 nm, and crystallinities larger than 140%. The above results indicate that the ZSM-35 molecular sieve provided by the present invention has the characteristics of small grain size and high crystallinity.

In Comparative Examples 4-6 and Examples 7-9, the isomerization catalysts were produced using the molecular sieves synthesized in Comparative Examples 1-3 and Examples 1-3, respectively. It should be particularly noted that the isomerization catalysts produced in the above Comparative Examples and Examples were produced essentially by the same method except for the different source of molecular sieves. Specifically, in Examples 10 and 11, the isomerization experiments were carried out using the above isomerization catalyst with etherified C5 components and etherified C4 components as the feedstock, respectively. Seen from the experimental data of Examples 10 and 11, the catalysts produced by the method of the present invention (Examples 7-9) exhibited higher isomerization activity and selectivity in the skeletal isomerization reaction for C5 and C4 light olefins compared to the conventional catalysts (Comparative Examples 4-6).

The difference between the production process of the isomerization catalysts produced in Examples 4-6 and that of Examples 7-9 is as follows: in the catalyst production process of Examples 4-6, a total of three times of baking steps were carried out, while there was only one baking step during the process of Examples 7-9. In other words, the template agent was not removed from the template agent-containing molecular sieve with small grains and high crystallinity used in Examples 7-9 prior to extrusion, and the molecular sieve was not baked after the ammonium exchange. Seen from the experimental data of Examples 10 and 11, the catalysts produced by the process involving one baking step (Examples 7-9) exhibited higher isomerization activity and selectivity in the skeletal isomerization reaction for C5 and C4 light olefins compared to the catalysts produced by the process involving three baking steps (Examples 4-6).

The above results indicate that the isomerization catalyst obtained by controlling the preparation conditions with the template agent-containing ZSM-35 molecular sieve as the feedstock can catalyze the conversion of light straight-chain olefins to light branched olefins, and the isomerization catalyst has higher catalytic activity and selectivity at lower temperatures.

Of course, the present invention may also have various other embodiments. A person skilled in the art may make various corresponding changes and modifications according to the present invention without departing from the spirit and essence of the present invention, but these corresponding changes and modifications shall all fall within the protection scope of the claims of the present invention.

**Claims**

1. A method for producing a ZSM-35 molecular sieve, comprising:

   mixing a silicon source, an aluminum source, an alkali, and a template agent with water to form a mixed solution, adding polyacrylamide to the mixed solution to form a feedstock solution, and
   crystallizing the feedstock solution at 140-160°C for 12-24 h and then at 120-140°C for 48-72 h to obtain the ZSM-35 molecular sieve,
   wherein the template agent comprises pyrrolidine.

2. The method according to claim 1, wherein the silicon source, the aluminum source, the alkali, pyrrolidine (Py) and water are mixed in molar ratios of $SiO_2/Al_2O_3$=20-110, $H_2O/SiO_2$=10-80, $NaOH/SiO_2$=0.1-1.0, and $Py/SiO_2$=0.1-1.0;

   the polyacrylamide is added in an amount of 0.1-1% by weight of the mixed solution;
   the silicon source comprises one or more of silica sol, solid silica gel and white carbon black;
   the aluminum source comprises one or more of aluminum sulfate, aluminum oxide and sodium meta-aluminate; and
   the alkali comprises sodium hydroxide.

3. The method according to claim 2, wherein the silicon source, the aluminum source, the alkali, pyrrolidine (Py) and water are mixed in molar ratios of $SiO_2/Al_2O_3$=30-90, $H_2O/SiO_2$=20-50, $NaOH/SiO_2$=0.2-0.5, and $Py/SiO_2$=0.2-0.5.

4. The method according to claim 2, wherein the polyacrylamide has a weight-average molecular weight of 3 million -10 million.

5. A ZSM-35 molecular sieve obtained by the method for producing a ZSM-35 molecular sieve according to any one of claims 1-4.

6. The ZSM-35 molecular sieve according to claim 5, further containing a template agent.

7. The ZSM-35 molecular sieve according to claim 5 or 6, which is lamellar crystal, and has a grain thickness of less than or equal to 50 nm, a grain length and/or the grain width of less than or equal to 500 nm, and a crystallinity of greater than or equal to 120%.

8. The ZSM-35 molecular sieve according to claim 7, having a grain thickness of less than 50 nm, a grain length and/or width of less than or equal to 200 nm, and a crystallinity of greater than or equal to 140%.

9. A method for producing an isomerization catalyst, comprising:

   subjecting a template agent-containing ZSM-35 molecular sieve to ammonium salt exchange,
   then mixing it with water, a binder, an acid and an extrusion aid, and
   then extruding, drying, and baking the mixture to obtain the isomerization catalyst,
   wherein the template agent-containing ZSM-35 molecular sieve is not subjected to a process of removing the template agent prior to the extrusion, and is not subjected to a baking process after the ammonium salt exchange.

10. The method according to claim 9, wherein the acid comprises an organic acid and/or an inorganic acid;

    the binder comprises one or more of alumina sol, alumina, and pseudo-boehmite;
    the extrusion aid comprises Sesbania powder and/or methyl cellulose; and
    the template agent-containing ZSM-35 molecular sieve comprises the ZSM-35 molecular sieve according to any one of claims 5-8.

11. The method according to claim 10, wherein the organic acid comprises acetic acid and/or citric acid; and the binder comprises pseudo-boehmite.

12. The method according to any one of claims 9-11, wherein the baking is carried out at a temperature of 530-550°C after the extrusion.

13. The method according to claim 12, wherein the baking is carried out for 4-8 h.

14. An isomerization catalyst obtained by the method for producing an isomerization catalyst according to any one of claims 9-13.

15. The isomerization catalyst according to claim 14, wherein the template agent-containing ZSM-35 molecular sieve in the isomerization catalyst accounts for 80% or more of the total mass of the isomerization catalyst.

16. The isomerization catalyst according to claim 15, wherein the template agent-containing ZSM-35 molecular sieve in the isomerization catalyst accounts for 90% or more of the total mass of the isomerization catalyst.

17. An isomerization method, comprising: catalyzing an isomerization reaction of light straight-chain olefins of C6 or less using the isomerization catalyst according to any one of claims 14-16 to convert them into isomeric light branched olefins of C6 or less, under a non-hydrogen exposure condition.

18. The isomerization method according to claim 17, wherein the isomerization reaction of the olefins is carried out at a pressure of 0.05-0.5 MPa, an LHSV of 2.0-6.0 $h^{-1}$ and a temperature of 240-420°C.

19. The isomerization method according to claim 18, wherein the isomerization reaction is carried out at a pressure of 0.1-0.3 MPa, an LHSV of 3.0-5.0 $h^{-1}$ and a temperature of 260-385°C.

20. The isomerization method according to any one of claims 17-19, wherein the light straight-chain olefins comprise one or more of n-butene, n-pentene, etherified C4 components, and etherified C5 components.

Fig. 1

a

b

Fig. 2

Reactor

N₂

Tile

Back pressure
valve

Wet type gas meter

Oil-feed pump

Pneumatic diaphragm valve

Knockout drum

Sampling port

Feedstock tank

Product tank

Fig. 3

## INTERNATIONAL SEARCH REPORT

| International application No. |
|---|
| **PCT/CN2022/090246** |

### A. CLASSIFICATION OF SUBJECT MATTER

C01B 39/44(2006.01)i; B01J 29/67(2006.01)i; C07C 5/27(2006.01)i

According to International Patent Classification (IPC) or to both national classification and IPC

### B. FIELDS SEARCHED

Minimum documentation searched (classification system followed by classification symbols)

C01B 39/-; B01J 29/-; C07C 5/-

Documentation searched other than minimum documentation to the extent that such documents are included in the fields searched

Electronic data base consulted during the international search (name of data base and, where practicable, search terms used)

CNABS, CNTXT, VEN, CNKI, WEB OF SCIENCE: ZSM-35, 沸石, 分子筛, FER, 镁碱, 聚丙烯酰胺, 分散, PAM, 吡咯烷, 异构, 两段, 分段, 两步, 分步, zeolite, molecular sieve, ferriirite, alkali-magnesium, magnesium-alkali, polyacrylamide, disperse, pyrrolidine, isomerization, isomerize, dual stage, two stage, multiple stage, dual step, two step, multiple step, stepwise

### C. DOCUMENTS CONSIDERED TO BE RELEVANT

| Category* | Citation of document, with indication, where appropriate, of the relevant passages | Relevant to claim No. |
|---|---|---|
| Y | CN 111392745 A (CHINA UNIVERSITY OF PETROLEUM-BEIJING) 10 July 2020 (2020-07-10) description, paragraphs [0006]-[0025] | 1-20 |
| Y | CN 101391780 A (YAO HUA) 25 March 2009 (2009-03-25) description, page 3, paragraph 3-page 4, paragraph 2 | 1-20 |
| Y | CN 108910910 A (CHINA UNIVERSITY OF PETROLEUM-BEIJING) 30 November 2018 (2018-11-30) description, paragraphs [0033]-[0035] | 1-20 |
| Y | CN 105983435 A (ZHUO RUNSHENG) 05 October 2016 (2016-10-05) description, paragraphs [0017]-[0036] | 1-20 |
| Y | CN 103073025 A (EAST CHINA NORMAL UNIVERSITY) 01 May 2013 (2013-05-01) description, paragraphs [0002], [0006] and [0009]-[0029] | 1-20 |
| Y | CN 104495869 A (PETROCHINA CO., LTD. et al.) 08 April 2015 (2015-04-08) description, paragraphs [0003], [0006] and [0007]-[0024] | 1-20 |

☑ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **11 July 2022** | **27 July 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| | International application No. |
|---|---|
| | **PCT/CN2022/090246** |

### C. DOCUMENTS CONSIDERED TO BE RELEVANT

| Category* | Citation of document, with indication, where appropriate, of the relevant passages | Relevant to claim No. |
|---|---|---|
| Y | CN 107010636 A (CHINA UNIVERSITY OF PETROLEUM-BEIJING) 04 August 2017 (2017-08-04) description, paragraphs [0011]-[0038] | 1-20 |
| Y | CN 105129813 A (EAST CHINA NORMAL UNIVERSITY) 09 December 2015 (2015-12-09) description, paragraphs [0002], [0003] and [0005]-[0011] | 1-20 |
| A | CN 110614121 A (DALIAN INSTITUTE OF CHEMICAL PHYSICS, CHINESE ACADEMY OF SCIENCES) 27 December 2019 (2019-12-27) entire document | 1-20 |
| A | US 4016245 A (MOBIL OIL CORP.) 05 April 1977 (1977-04-05) entire document | 1-20 |
| Y | 王洁等 (WANG, Jie et al.). "无模板变温两段晶化法ZSM-5分子筛的制备与表征 (Template-free synthesis and characterization of ZSM-5 molecular sieves by two-step method with temperature change)" 天然气化工 (C1化学与化工) (Natural Gas Chemical Industry (C1 Chemistry and Chemical Engineering)), Vol. 45, No. 1, 31 December 2020 (2020-12-31), pages 17-21 | 1-20 |
| A | 卢永斌等 (LU, Yongbin et al.). "两段晶化法快速合成均匀单分散小晶粒ZSM-5分子筛 (Rapid synthesis of ZSM-5 zeolite with small-sized and single dispersed crystals by two-stage crystallzation)" 天然气化工 (C1化学与化工) (Natural Gas Chemical Industry (C1 Chemistry and Chemical Engineering)), Vol. 42, No. 3, 31 December 2017 (2017-12-31), pages 1-6 | 1-20 |
| A | SANG, Shiyun et al. "Synthesis of small crystals zeolite NaY" Materials Letters, Vol. 60, 31 December 2006 (2006-12-31), pages 1131-1133 | 1-20 |

Form PCT/ISA/210 (second sheet) (January 2015)

## INTERNATIONAL SEARCH REPORT
### Information on patent family members

International application No.

**PCT/CN2022/090246**

| Patent document cited in search report | | | Publication date (day/month/year) | Patent family member(s) | | | Publication date (day/month/year) |
|---|---|---|---|---|---|---|---|
| CN | 111392745 | A | 10 July 2020 | None | | | |
| CN | 101391780 | A | 25 March 2009 | None | | | |
| CN | 108910910 | A | 30 November 2018 | US | 2020038848 | A1 | 06 February 2020 |
| CN | 105983435 | A | 05 October 2016 | None | | | |
| CN | 103073025 | A | 01 May 2013 | None | | | |
| CN | 104495869 | A | 08 April 2015 | None | | | |
| CN | 107010636 | A | 04 August 2017 | None | | | |
| CN | 105129813 | A | 09 December 2015 | None | | | |
| CN | 110614121 | A | 27 December 2019 | None | | | |
| US | 4016245 | A | 05 April 1977 | None | | | |

Form PCT/ISA/210 (patent family annex) (January 2015)